(19) Europäisches Patentamt
European Patent Office
Office européen des brevets

(11) Veröffentlichungsnummer : **0 468 927 A2**

# (12) EUROPÄISCHE PATENTANMELDUNG

(21) Anmeldenummer : **91810582.6**

(22) Anmeldetag : **18.07.91**

(51) Int. Cl.$^5$ : **C07C 69/635,** C07C 233/15, A01N 37/02, A01N 37/22, C07C 53/46, C07C 67/14, C07C 67/08, C07C 67/347, C07C 53/23, C07C 323/20, C07D 213/647

(30) Priorität : **27.07.90 CH 2496/90**

(43) Veröffentlichungstag der Anmeldung :
**29.01.92 Patentblatt 92/05**

(84) Benannte Vertragsstaaten :
**BE CH DE DK ES FR GB GR IT LI LU NL**

(71) Anmelder : **CIBA-GEIGY AG**
**Klybeckstrasse 141**
**CH-4002 Basel (CH)**

(72) Erfinder : **Böger, Manfred**
**Wilhelm Glockstrasse 14**
**W-7858 Weil am Rhein 5 (DE)**
Erfinder : **Maienfisch, Peter, Dr.**
**Aegertenstrasse 21**
**CH-4118 Rodersdorf (CH)**

## (54) Carboxymethylcyclopropanderivate.

(57) Neue 2-(2-Chlor-2-fluorcyclopropyl)-essigsäurederivate der Formel I

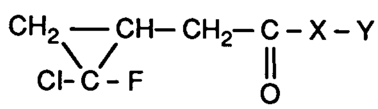

(I)

worin
X Sauerstoff oder -NR-,
Y Wasserstoff oder jeweils unsubsrituiertes oder subsrituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl, und
R Wasserstoff oder $C_1$-$C_6$-Alkyl bedeuten,
können als Schädlingsbekämpfungsmittel eingesetzt werden. Vorzugsweise können Insekten und Arachniden bekämpft werden.

EP 0 468 927 A2

Die vorliegende Erfindung betrifft neue Derivate von 2-(2-Chlor-2-fluorcyclopropyl)-essigsäure, Verfarhen und Zwischenprodukte zu ihrer Herstellung, Schädlingsbekämpfungsmittel, welche diese Verbindungen enthalten, sowie ihre Verwendung bei der Kontrolle von Schädlingen.

Die erfindungsgemässen 2-(2-Chlor-2-fluorcyclopropyl)-essigsäurederivate entsprechen der Formel I

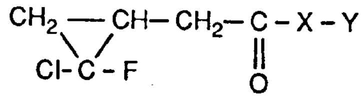

(I)

worin

X Sauerstoff oder -NR-,

Y Wasserstoff oder jeweils unsubstituiertes oder substituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl, und

R Wasserstoff oder $C_1$-$C_6$-Alkyl bedeuten.

Vorzugsweise steht Y im Rahmen der vorliegenden Erfindung für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Aryl, $C_3$-$C_{20}$-Halogenalkenyl, $C_3$-$C_{20}$-Halogenalkinyl; durch Halogen oder $C_1$-$C_4$-Alkyl subsbtuiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyan, Benzoyl, Halogenbenzoyl, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, Phenylthio oder Halobenphenylthio substituiertes Aryl; oder durch Hydroxy, Halogen, Di-$C_1$-$C_4$-alkylamino, $C_1$-$C_4$-Alkoxy, $C_1$-$C_4$-Halogenalkoxy, Cyan, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_1$-$C_6$-Alkoxycarbonyloxy, $C_3$-$C_7$-Cycloalkyl, Aryl, Aryloxy, Anilinocarbonyloxy, Benzyloxy, Arylcarbonyloxy, Arylthio, Arylsulfonyl, Arylsulfinyl, Arylsulfonyloxy, Arylcarbonyl oder Pyridyl substituiertes $C_1$-$C_{20}$-Alkyl; wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Benzyloxy, Cyan, Phenoxy, Halogenphenoxy, Pyridyloxy, Halogenpyridyloxy, $C_1$-$C_4$-Halogenalkylpyridyloxy, $C_1$-$C_4$-Alkylthiadiazolyloxy, Phenylthio oder Halogenphenylthio subsbtuiert sein können, steht.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkylthio, Halogenalkoxy, Halogenphenylthio oder Halogenphenoxy.

Die als Substituenten in Betracht kommenden Alkyl-, Alkylthio-, Alkoxyalkoxy- und Alkoxyreste können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl, Decyl, Dodecyl und ihre Isomeren genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio.

Sind die als Substituenten in Betracht kommenden Alkyl-, Alkoxy-, Alkenyl-, Alkinyloder Arylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise $CHF_2$ oder $CF_3$; das ein- bis fünffach durch Fluor, Chlor und/oder Brom substituierte Aethyl wie zum Beispiel $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie z.B. $CH_2CHBrCH_2Br$, $CF_2CHFCF_3$, $CF_2CF_2CF_3$ oder $CH(CF_3)_2$; das ein- bis neunfach durch Fluor, Chlor und/oder Brom substituierte Butyl oder eines seiner Isomeren wie zum Beispiel $CF(CF_3)CHFCF_3$ oder $CH_2(CF_2)_2CF_3$.

Sind die unter Y definierten Alkyl-, Cycloalkyl- oder Arylgruppen durch andere Substituenten substituiert, so können sie ein- oder mehrfach durch den gleichen oder verschiedene der aufgezählten Substituenten substituiert sein. Vorzugsweise sind in dem substituierten Gruppen ein oder zwei weitere Substituenten vorhanden.

Bei den als Substituenten in Betracht kommenden Cycloalkylresten handelt es sich beispielsweise um Cyclopropyl, Cyclobutyl, Cyclopentyl oder Cyclohexyl.

Alkenyl- und Alkinylgruppen enthalten eine oder mehrere, vorzugsweise nicht mehr als drei, ungesättigte Kohlenstoff-Kohlenstoff-Bindungen. Die Doppel- oder Dreifachbindungen sind von der Verknüpfungsstelle zur

Brücke X durch mindestens ein gesättigtes Kohlenstoffatom getrennt. Typische Vertreter sind Allyl, Methalllyl, 2-Butenyl, 3-Butenyl, Propargyl, 2-Butinyl oder 3-Butinyl.

Aryl steht für einen aromatischen Kohlenwasserstoffrest. Vorzugsweise wird unter Aryl Phenyl oder Naphthyl verstanden.

Beispiele für Alkoxyalkoxyreste sind Methoxymethoxy, Methoxyäthoxy, Aethoxyäthoxy, Aethoxymethoxy, Propoxymethoxy, Propoxyäthoxy, Methoxypropoxy, Butoxymethoxy oder Propoxyäthoxy.

Beispiele für Alkoxycarbonyreste sind Methoxycarbonyl, Aethoxycarbonyl, Propoxycarbonyl, Isopropoxycarbonyl oder Butoxycarbonyl. Alkylcarbonyl steht beispielsweise für Acetyl, Propionyl, Butyl oder Valeryl, sowie Isomere davon. Alkylcarbonyloxy steht zum Beispiel für Acetoxy, Propionyloxy oder Butyryloxy.

Aus der Literatur sind 2,2-Difluorcyclopropyläthanderivate als Schädlingsbekämpfungsmittel aus der EP-A-318425 bekannt. Diese Verbindungen können jedoch als Schädlingsbekämpfungsmittel nicht immer voll befriedigen. Daher besteht weiterhin ein Bedürfnis nach Wirkstoffen zur Schädlingskontrolle mit verbesserten Eigenschaften.

Gemäss vorliegender Erfindung werden daher die Verbindungen der Formel I zur Schädlingsbekämpfung, insbesondere von Insekten und Vertretern der Klasse der Akarina vorgeschlagen.

Wegen ihrer vorteilhaften biologischen Wirkung sind unter den Verbindungen der Formel I solche Untergruppen herauszuheben, in denen entweder

a) X Sauerstoff, -NH-, $-NCH_3-$ oder $-NC_2H_5-$ bedeutet, oder

b) Y für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Phenyl, Naphthyl, $C_3$-$C_{20}$-Halogenalkenyl, $C_3$-$C_{20}$-Halogenalkinyl; durch Fluor, Chlor, Brom oder $C_1$-$C_3$-Alkyl substituiertes $C_3$-$C_7$-Cycloalkyl; durch Fluor, Chlor, Brom oder $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl oder Naphthyl, oder durch Hydroxy, Fluor, Chlor, Brom, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Phenyl, Phenoxy, Phenylthio, Phenylsulfonyloxy oder Pyridyl subsbtuiertes $C_1$-$C_{20}$-Alkyl, wobei die Phenyl- und Pydylgruppen jeweils durch Muor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiert sein können, steht.

Von den Verbindungen der Untergruppe a) sind solche bevorzugt, worin X Sauerstoff oder -NH- bedeutet.

Aus der Untergruppe b) der Verbindungen der Formel I sind einerseits diejenigen herauszustellen, worin Y Phenyl, Benzyl, Naphthyl, 3-Pydylmethyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl, Benzyl, Naphthyl oder 3-Pydylmethyl bedeutet. Von diesen Verbindungen sind wiederum solche bevorzugt, worin X Sauerstoff oder -NR- und R Wasserstoff, Methyl oder Aethyl bedeuten.

Andererseits sind unter der Untergruppe b) der Verbindungen der Formel I auch diejenigen besonders hervorzuheben, worin Y $C_1$-$C_{12}$-Alkyl oder durch Hydroxy, Fluor, Chlor, Brom, Dimethylamino, Methoxy, Aethoxy, Methoxyäthoxy, Aethoxyäthoxy, Methylthio, Aethylthio, Cyclopropyl, Cyclopentyl, Cyclohexyl, Phenyl oder Phenoxy substituiertes $C_1$-$C_{12}$-Alkyl bedeutet, wobei der Phenyl- oder Phenoxyrest jeweils durch Fluor, Chlor, Brom, Phenoxy, Halogenphenoxy oder Phenylthio substituiert sein kann. Von dieser Gruppe von Verbindungen sind solche bevorzugt, worin X Sauerstoff oder -NR- und R Wasserstoff oder $C_1$-$C_4$-Alkyl bedeuten.

Eine sehr interessante Gruppe von Wirkstoffen aus der Untergruppe b) der Formel I zeichnet sich dadurch aus, dass Y $C_3$-$C_{12}$-Alkenyl, $C_3$-$C_{12}$-Alkinyl oder jeweils durch Fluor, Chlor oder Brom subsbtuiertes $C_3$-$C_{12}$-Alkenyl oder $C_3$-$C_{12}$-Alkyl bedeutet. Aus dieser Gruppe geniessen solche Verbindungen eine Vorzugsstellung, worin X Sauerstoff oder -NR- und R Wasserstoff oder $C_1$-$C_4$-Alkyl bedeuten.

Besonders hervorzuheben ist auch diejenige Gruppe von Verbindungen der Formel I worin der Rest Y durch das folgende Formelfragment wiedergegeben ist:

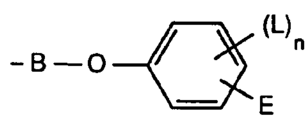

worin

B für eine $C_2$-$C_6$-Alkylenbrücke,

L für Halogen oder Methyl,

n für die Zahl null, eins oder zwei und

E für Benzyloxy, Phenyl, Halogenphenoxy, Naphthoxy, Pyridyloxy, Halogenpyridyloxy, $C_1$-$C_4$-Halogenalkylpyridyloxy, $C_1$-$C_4$-Alkylthiadiazolyloxy, Phenylthio oder Halogenphenylthio stehen.

Das Radikal E soll dabei beispielsweise die folgenden Ausführungsformen annehmen können:
Phenoxy,
Phenylthio,
3,5-Dichlorpyrid-2-yloxy,
2-Pyridyloxy,
Benzyloxy,
3-Chlorphenoxy,
3-Methyl- 1,2,4-thiadiazol-5-yloxy,
4-Fluorphenoxy,
3-Fluorphenoxy,
2-Fluorphenoxy,
3,5-Difluorphenoxy,
3-Isopropyl-1,2,4-thiadiazol-5-yloxy,
4-Chlorphenoxy,
2-Chlorphenoxy,
5-Trifluormethyl-pyrid-2-yloxy,
5-(2,2-Dichlor-1,1,2-trifluoräthyl)-pyrid-2-yloxy,
4-Chlorphenylthio,
3,4-Dichlorphenylthio und
2-Naphthoxy.

Bevorzugt sind insbesondere auch solche Verbindungen der Formel 1, worin X für Sauerstoff und der Rest Y für das Formelfragment

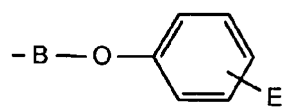

stehen, worin

B eine Brücke 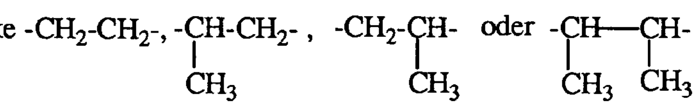 und

E Phenoxy, Phenylthio oder Halogenphenoxy bedeuten.

Als bevorzugte erfindungsgemässe Einzelverbindungen der Formel I sind zu nennen:
2-(2-Chlor-2-fluorcyclopropyl)-essigsäure,
2-(2-Chlor-2-fluorcyclopropyl)-essigsäurebenzylester,
2-(2-Chlor-2-fluorcyclopropyl)-essigsäure-2-(4-chlor-phenoxy)-äthylester,
2-(2-Chlor-2-fluorcyclopropyl)-essigsäure-2-(4-phenoxy-phenoxy)-äthylester und
2-(2-Chlor-2-fluorcyclopropyl)-essigsäure-2-(4-phenylthio-phenoxy)-äthylester.

Die erfindungsgemässen 2-(2-Chlor-2-fluorcyclopropyl)-essigsäurederivate der Formel I lassen sich herstellen, indem man entweder

a) ein 2-(2-Chlor-2-fluorcyclopropyl)-essigsäurehalogenid der Formel II

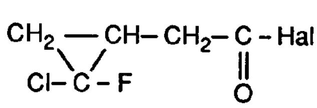 (II)

worin Hal für Chlor oder Brom steht, gegebenenfalls in einem inerten Lösungsmittel und in Gegenwart eines säurebindenden Mittels mit einem Alkohol oder Amin der Formel III

H-X-Y (III)

worin X und Y die unter Formel I gegebenen Bedeutungen haben, umsetzt, oder

b) ein 3-Butensäurederivat der Formel IV

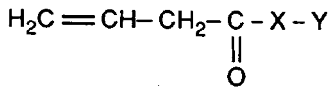

(IV)

worin X und Y die unter Formel I gegebenen Bedeutungen haben, in einem inerten Lösungsmittel mit Chlorfluorcarben umsetzt, oder

c) die freie 2-(2-Chlor-2-fluorcyclopropyl)-essigsäure der Formel Ia

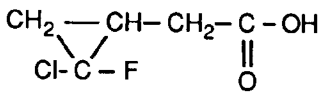

(Ia)

gegebenenfalls in Gegenwart eines inerten Lösungsmittels und eines Katalysators oder eines wasserentziehenden Mittels mit einem Alkohol oder Amin der Formel III umsetzt.

Die Umsetzung des Verfarhens a) (II + III→I) erfolgt vorzugsweise in einem inerten, hydroxylgruppenfreien Lösungsmittel in Anwesenheit einer organischen Base, wie zum Beispiel Pyridin, 4-Dimethylaminopyridin, Lutidin, Collidin, Trialkylamin, N,N-Dialkylanilin, oder einer bicyclischen, nicht nucleophilen Base wie 1,4-Diazabicyclo[2.2.2] octan (DABCO), 1,5-Diazabicyclo[4.3.0]non-5-en (DBN) oder 1,8-Diazabicyclo[5.4.0]undec-7-en (1,5-5) (DBU). Die Reaktion wird im allgemeinen bei Temperaturen von -30°C bis +70°C, vorzugsweise von -10°C bis +50°C durchgeführt. Man arbeitet dabei zweckmässigerweise in Gegenwart eines reaktionsinerten Lösungsmittels oder Lösungsmittelgemisches. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenwasserstoff, Tetrachloräthylen; Aether und ätherartige Verbindungen wie Dialkyläther (Diäthyläther, Diisopropyläther, tert-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; Ketone wie Aceton, Diäthylketon, Methyläthylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen, oder im Falle, dass die Verbindung der Formel III ein Amin darstellt (X = NR), kann anstelle der Base auch ein zweites Aequivalent oder auch ein grösserer Ueberschuss der Verbindung der Formel III eingesetzt werden. Die Umsetzung wird unter dem Druck der Umgebung durchgeführt, wenngleich sie auch bei erhöhtem oder vermindertem Druck durchgeführt werden könnte.

Für die Durchführung der Reakbonsvariante b) (IV + Chlorfluorcarben → I) eignen sich als Lösungsmittel bevorzugt Aether wie Diglyme, Triglyme oder Tetraglyme. Die Erzeugung von Chlorfluorcarben erfolgt nach aus der Fachliteratur an sich bekannten Methoden (Burton und Hahnfeld, Fluorine Chem. Rev. 8 (1977), 119ff.). Als Chlorfluorcarbenspender eignen sich beispielsweise Alkalimetalldichlorfluoracetate wie Natriumdichlorfluoracetat oder Halogenchlorfluorkohlenwasserstoffe wie Dichlorfluormethan.

Bei der Verfahrensvariante c) (Ia + III → I) wird die Reaktion vorteilhafterweise in Gegenwart von für Veresterungen üblichen, wasserabspaltenden Reagenzien durchgefürt, wie zum Beispiel in Anwesenheit eines Carbodiimids [Dicyclohexylcarbodiimid (DCC)] oder eines 1 -Alkyl-2-halogen-pyridiniumsalzes wie 1-Methyl-2-chlorpyridiniumjodid. Zweckmässigerweise wird die Reaktion dann in Gegenwart eines reaktionsinerten Lösungsmittels oder Lösungsmittelgemisches bei Temperaturen von -30°C bis +70°C, vorzugsweise -10°C bis +50°C durchgeführt. Man arbeitet bevorzugt in Gegenwart einer Base wie beispielsweise in Gegenwart eines organischen Amins wie eines Trialkylamins (Trimethylamin, Triäthylamin, Tripropylamin oder Düsopropyläthylamin), eines Pyridins (Pyridin selbst, 4-Dimethylaminopyridin oder 4-Pyrrolidinopyridin), eines Morpholins (N-Methylmorpholin) oder eines N,N-Dialkylanilins (N,N-Dimethylanilin oder N-Methyl-N-äthylanilin). Als Lösungsmittel eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther), tert.-Butylmethyläther usw.), Anisol, Dioxan, Tetrahydrofuran; Nitrile wie Acetonitril, Propionitril; Ester wie Aethylacetat (Essigsäureäthylester), Propylacetat oder Butylacetat; und Gemische solcher Lösungsmittel untereinander.

Stellt die Verbindung der Formel III einen Alkohol dar (X = O) so kann die Verfahrensvariante c) auch in Gegenwart eines Säurekatalysators, wie zum Beispiel $H_2SO_4$, HCl oder einer Sulfonsäure wie Methansulfonsäure oder p-Toluolsulfonsäure, durchgeführt werden. Man arbeitet dabei vorteilhafterweise mit einem Ueberschuss des Alkohols der Formel III. Bei diesem Verfahren kann freiwerdendes Wasser kontinuierlich aus dem

Reaktionsgemisch entfernt werden. Eine übliche Methode dazu ist das Entfemen des Reaktionsproduktes Wasser durch Abdestillieren eines Azeotrops des Lösungsmittels mit Wasser. Dazu geeignete Lösungsmittel sind Benzol, Toluol, Xylol, Methylenchlorid oder Chloroform.

Grundsätzlich sind die verschiedenen Derivate der Formel I auch aus Umesterung oder Amidierung aus den leicht zugänglichen Niederalkylestem der 2-(2-Chlor-2-fluorcyclopropyl)-essigsäure erhältlich.

Beispielsweise können die Derivate des Estertyps der Formel I (X = O) durch basen- oder säurekatalysierte Umesterung der Niederalkylester der Formel Ib

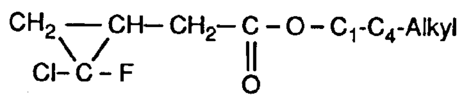

(Ib)

mit den Alkoholen der Formel IIIa

H-O-X-Y (IIIa)

worin X und Y die unter Formel I gegebenen Bedeutungen haben, erhalten werden. Als Säurekatalysatoren besonders geeignet sind HCl, $H_2SO_4$ oder eine Sulfonsäure. Bei der basenkatalysierten Umesterung verwendet man vorzugsweise als Base das Natrium- oder Kaliumalkoholat des Alkohols der Formel IIIa, welches aus IIIa beispielsweise durch Zugabe von Natrium- oder Kaliumhydrid zugänglich sind. Die Umesterungsreaktion wird vorzugsweise bei Temperaturen zwischen -20°C und + 120°C, insbesondere zwischen 0°C und +100°C durchgeführt. Die Alkoholkomponente IIIa wird mit Vorteil im Ueberschuss verwendet. Als Lösungsmittel kommen Aether, wie Diäthyläther, Diisopropyläther, Dioxan oder Tetrahydrofuran, halogenierte Kohlenwasserstoffe oder aliphatische oder aromatische Kohlenwasserstoffe in Frage.

Derivate des Amidtyps der Formel I (X = NR) werden aus den Niederalkylestern der Formel Ib erhalten, indem man diese mit Aminen der Formel IIIb

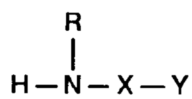

(IIIb)

worin R, X und Y die unter Formel I gegebenen Bedeutungen haben, umsetzt. Die Amidierungsreaktionen führt man zwischen Temperaturen zwischen 0°C und + 120°C durch. Mit Vorteil werden die Reaktanden in einem inerten Lösungsmittel oder Lösungsmittelgemisch umgesetzt. Es eignen sich hierfür beispielsweise aliphatische und aromatische Kohlenwasserstoffe wie Benzol, Toluol, Xylole, Petroläther, Hexan; halogenierte Kohlenwasserstoffe wie Chlorbenzol, Methylenchlorid, Aethylenchlorid, Chloroform, Tetrachlorkohlenstoff, Tetrachloräthylen; Aether und ätherartige Verbindungen wie Dialkyläther (Diäthyläther, Diisopropyläther, tert.-Butylmethyläther usw.), Anisol, Dioxan, Tetrahydrofuran; Nitrile wie Acetonitril, Propionitril; Alkohole wie Methanol, Aethanol, Propanol, Isopropanol; oder Wasser. Die Aminkomponente IIIb wird mit Vorteil im Ueberschuss verwendet.

Verschiedene Verbindungen der Formeln III, IIIa und IIIb und IV und ihre Herstellung sind aus der Literatur bekannt und zum Teil im Handel erhältlich. Die noch nicht in der Literatur beschriebenen Verbindungen der Formeln III, IIIa, IIIb und IV können analog zu den bekannten Verfahren nach üblichen Synthesemethoden erhalten werden.

Die Verbindungen der Formel IV können aus bekannten Produkten erhalten werden, indem man 3-Butensäure der Formel V

$CH_2 = CH\text{-}CH_2\text{-}COOH$ (V)

gegebenenfalls in Gegenwart eines inerten Lösungsmittels und eines Katalysators oder eines wasserentziehenden Mittels mit einem Alkohol oder Amin der Formel III umsetzt.

Die Reaktionsbedingungen dieser Veresterung oder Amidierung entsprechen denen der Verfahrensvariante c) des erfindungsgemässen Verfahrens zur Herstellung der Verbindungen der Formel I.

Die Verbindung der Formel II ist neu. Sie wurde speziell für die Synthese der Wirkstoffe der Formel I entwickelt. Die Verbindung der Formel II bildet daher einen Bestandteil der vorliegenden Erfindung.

Die Verbindungen der Formeln Ia und II werden in einfacher Weise aus den leicht zugänglichen Estern der Formel Ib erhalten, indem man letztere in üblicher Weise hydrolysiert und halogeniert. In gleicher Weise kann man die Verbindungen der Formeln Ia und II auch aus anderen einfachen Estern, wie zum Beispiel dem 2-(2-Chlor-2-fluorcyclopropyl)-essigsäurebenzylester, erhalten. Die Bedingungen zur Synthese der freien Säure der Formel Ia aus dem zugrundeliegenden Benzylester oder einem Ester der Formel Ib und die nach-

folgende fakultative Ueberführung dieser Säure Ia in das Säurehalogenid der Formel II entsprechen den üblichen Bedingungen für eine säure- oder basekatalysierte Hydrolyse, beziehungsweise für die Halogenierung einer Carbonsäure.

Die Ester der Formel Ib und der entsprechende Benzylester lassen sich durch Addibon von Chlorfluorcarben an 3-Butensäureniederalylester ($C_1$-$C_4$) oder 3-Butensäurebenzylester einfach herstellen. Die Reaktionsbedingungen entsprechen denen der Reaktionsvariante b) für die Chlorfluorcarbenaddition.

Sofern nicht speziell erwähnt, liegen die Verbindungen der Formel I als Gemisch von zwei diastereomeren Enantiomerenpaaren vor. Die reinen Enantiomerenpaare erhält man mittels üblicher physikalischer Trennmethoden. Die optisch reinen Isomeren können erhalten werden, indem man von optisch reinen Ausgangsprodukten, wie der RR-, RS-, SR- oder SS-2-(2-Chlor-2-fluorcyclopropyl)-essigsäure, oder deren Halogeniden ausgeht, oder indem man die optisch reinen Isomeren aus den Enantiomerenpaaren (Racemate) nach an sich bekannten Methoden abtrennt.

Beispielsweise erhält man die optischen Isomeren der freien Säuren der allgemeinen Formel Ia durch Umsetzen von Verbindungen der allgemeinen Formeln II oder Ia mit einem chiralen Hilfsreagenz, wie zum Beispiel einem optisch aktiven Amin oder einem optisch aktiven Alkohol und anschliessender Trennung der so erhaltenen Diastereomeren mit Hilfe physikalischer Methoden (Tetrahedron 33, 2725 (1977)), wie Kristallisation, Destillation oder Fest-Flüssig-Chromatographie und anschliessender hydrolytischer Spaltung der erhaltenen reinen Diastereomeren, die entweder säure- oder basenkatalytisch geführt werden kann. Die erfindungsgemässen Verbindungen der Formel I können in optisch reiner Form nach Verfahrensvariante c) aus den optischen Isomeren der freien Säure erhalten werden.

Weiterhin können die bei der Synthese entstehenden Gemische optischer Isomere der allgemeinen Formel I durch Chromatographie an chiralen stationären Phasen, wie beispielsweise an Cyclodextrinen, Stärke oder an Polymere gebundene optisch aktive Aminosäurederivate, in die Enantiomere getrennt werden (Angew. Chem. 92, 14 (1980)).

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:

aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrobca spp., Epilachna spp., Eremnus spp., Leptinotarsa decemlineata, Lissorhoptrus spp. Melolontha spp., Orycaephilus spp., Oborhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxera spp.;
aus der Ordnung der Mallophaga zum Beispiel Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;

aus der Ordnung der Heteroptera zum Beispiel
Cimex spp., Distanbella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium comi, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gaströphilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.; und
aus der Ordnung der Thysanura zum Beispiel
Lepisma saccharina.

Insbesondere eignen sich die Verbindungen der Formel I in besonderem Masse zur Kontrolle der Schädlinge in Obst- und Gemüsekulturen, wie Spinnmilben und Blattläusen Weiter können in vorteilhafter Weise die Warmblüterparasiten wie Milben und Zecken mit den Wirkstoffen der Formel I bekämpft werden.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden und/oder Akariziden wesentlich verbreitem und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vemebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckrnitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, und Glykole sowie deren Aether und Ester, wie Propylenglykol, Dipropylenglykoläther, Aethylenglykol, Aethylenglykolmonomethyl- oder -äthyläther, Ketone, wie Cyclo- hexanon, Isophoron oder Diacetanolalkohol, starke polare Lösungsmittel, wie N-Methyl-2-pyrrolidon, Dimethylsulfoxid oder Dimethylformamid oder Wasser, Pflanzenöle, wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und-/oder anionaktive Tenside mit guten Emulgier-, Dispergier- und Netzeigenschaften in Betracht. Unter Tensiden sind auch Tensidgemische zü verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls subsbtuierten Ammoniumsalze von höheren Fettsären ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecyl-schwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichbonische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quartemäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammonium chlorid oder das Benzyl-di-(2-chloräthyl)-äthylammoniumbromid.

Die in der Formulierungstechnik gebräuchlichen Tenside sind beispielsweise in folgenden Publikationen beschrieben:

"Mc Cutcheon's Detergents and Emulsifiers Annual", Mc Publishing Corp., Glen Rock, NJ, USA, 1988",
H. Stache, "Tensid-Taschenbuch", 2. Aufl., C. Hanser Verlag München, Wien 1981.
M. and J. Ash. "Encyclopedia of Surfactants", Vol. I-III, Chemical Publishing Co., New York, 1980- 1981.

Die pesbziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrabonen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen: (% = Gewichtsprozent)

Emulgierbare Konzentrate:

| | |
|---|---|
| Aktiver Wirkstoff: | 1 bis 90 %, bevorzugt 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspension-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Herstellungsbeispiele

Beispiel H1: 2-(2-Chlor-2-fluorcyclopropyl)-essigsäurebenzylester

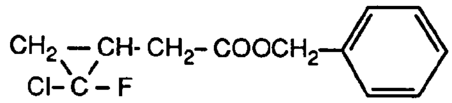

10,6 g 3-Butensäurebenzylester werden in 40 ml Diäthylenglykoldimethyläther (Diglyme) gelöst und bei +165°C innerhalb von 4 Stunden mit einer Lösung von 20,3 g Natriumdichlorfluoracetat in 40 ml Diglyme versetzt. Es wird für weitere 4 Stunden bei +165°C gerührt und anschliessend abgekühlt. Das ausgefallene Natriumchlorid

wird abgetrennt und mit wenig Diglyme nachgewaschen. Im Hochvakuum wird das Lösungsmittel bei +40°C abgedampft, der Rückstand in 80 ml Diäthyläther aufgenominen und zweimal mit je 20 ml Wasser gewaschen, über Natriumsulfat getrocknet und eingedampft. Danach wird der ölige Rückstand im Hochvakuum fraktioniert. Man erhält so den 2-(2-Chlor-2-fluorcyclopropyl)-essigsäurebenzylester als farblose Füssigkeit, Sdp. 110°C/0,25 mbar, $n_D^{20}$: 1,5031.

Beispiel H2: 2-(2-Chlor-2-fluorcyclopropyl)-essigsäure

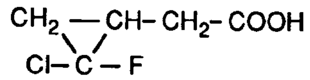

Zu einer Lösung von 1,85 g Kaliumhydroxid in 25 ml Methanol lässt man unter Eiskühlung 8,0 g 2-(2-Chlor-2-fluorcyclopropyl)-essigsäurebenzylester zutropfen und rührt für 4 Stunden bei Raumtemperatur. Anschliessend wird das Lösungsmittel abgedampft, der Rückstand mit 30 ml Aether versetzt und das ausgefallene Kaliumsalz abgetrennt. Das Salz wird in 20 ml Wasser gelöst, mit 3,5 ml konz. HCl angesäuert und dreimal mit je 20 ml Essigester extrahiert. Die vereinigte Essigesterphase wird zweimal mit je 10 ml Sole gewaschen, über Natriumsulfat getrocknet und eingedampft. Man erhält so die 2-(2-Chlor-2-fluorcyclopropyl)-essigsäure als gelbliches Oel mit einem Brechungsindex von $n_D^{20}$: 1,4442.

Beispiel H3: 2-(2-Chlor-2-fluorcyclopropyl)-essigsäure-2-(4-phenoxy-phenoxy)-äthylester

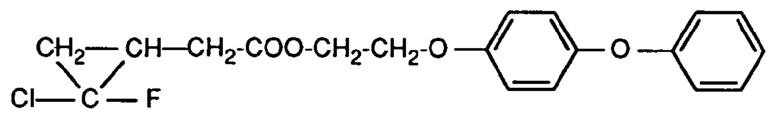

Zu einer Lösung von 1,0 g 2-(2-Chlor-2-fluorcyclopropyl)-essigsäure in 20 ml Dichlormethan wird 50 mg 4-Pyrrolidino-pyridin und 1,51 g 2-(4-Phenoxyphenoxy)-äthanol gegeben und auf 0°C abgekühlt. Bei einer Temperatur zwischen 0°C und +5°C wird portionsweise 1,50 g N,N-Dicyclohexylcarbodiimid zugegeben und anschliessend wird das Reaktionsgemisch bei Raumtemperatur für 5 Stunden gerührt. Der ausgefallene N,N-Dicyclohexyl-harnstoff wird abfiltriert und das Filtrat zweimal mit je 10 ml Wasser gewaschen, getrocknet und eingedampft. Der Rückstand wird durch Säulenchromatographie an Kieselgel (Laufmittel: Hexan-Essigester, 9:1) gereinigt. Man erhält so 2-(2-Chlor-2-fluorcyclopropyl)-essigsäure-2-(4-phenoxyphenoxy)-äthylester als leicht gelbliches Oel mit einem Brechungsindex von $n_D^{20}$: 1,5461.

Beispiel H4: 2-(2-Chlor-2-fluorcyclopropyl)-essigsäurechlorid

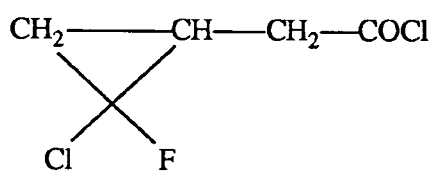

Zu einer Lösung von 1,53 g 2-(2-Chlor-2-fluorcyclopropyl)-essigsäure in 25 ml Tetrahydrofuran und 1 Tropfen Dimethylformamid wird 1,42 g Oxalylchlorid zugegeben und das Gemisch 1 Stunde bei Raumtemperatur nachgerührt. Danach wird das Reaktionsgemisch bei 80 mbar und +50°C Badtemperatur am Rotationsverdampfer eingedampft.

Man erhält so das 2-(2-Chlor-2-fluorcyclopropyl)-essigsäurechlorid als gelbliche Flüssigkeit.

Beispiel H5: 2-(2-Chlor-2-fluorcyclopropyl)-essigsäure-4-chloranilid

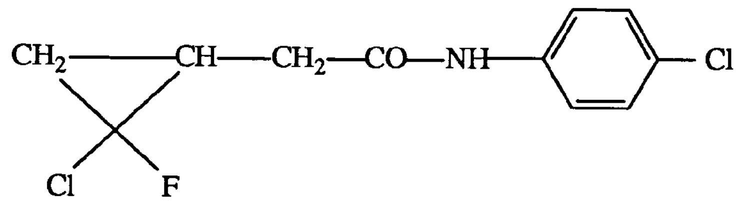

Das nicht weiter gereinigte im Beispiel H4 erhaltene 2-(2-Chlor-2-fluorcyclopropyl)-essigsäwechlorid wird in 40 ml Toluol aufgenommen und bei 0°C bis +5°C langsam eine Lösung von 1,02 g 4-Chloranilin und 0,95 ml Pyridin in 10 ml Toluol zugetropft. Das Reaktionsgemisch wird 2 Stunden bei Raumtemperatur nachgerührt, dann dreimal mit je 10 ml Sole gewaschen, über Natriumsulfat getrocknet und eingedampft. Der Rückstand wird aus einem Essigsäureäthylester/Hexan-Gemisch umkristallisiert.

Man erhält so das 2-(2-Chlor-2-fluorcyclopropyl)-essigsäure-4-chloranilid als hellbeiges Pulver mit einem Schmelzpunkt von 126-128°C.

Beispiel H-6: 2-(2-Chlor-2-fluorcyclopropyl)-essigsäure-2-(4-phenoxy-phenoxy)-äthyl-ester

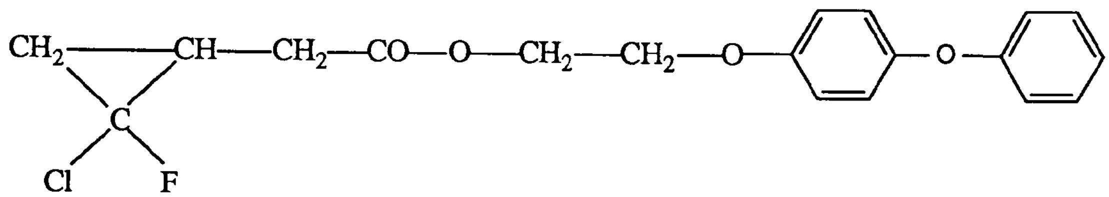

a) 3-Butensäure-2-(4-phenoxy-phenoxy)-äthylester

Zu einer Lösung von 6,45 g 3-Butensäure in 100 ml Dichlormethan setzt man unter Rühren bei Raumtemperatur 0,05 g 4-Pyrrolidino-pyridin und 17,25 g 2-(4-Phenoxy-phenoxy)-äthanol zu. Bei einer Temperatur von 0°C werden danach portionenweise 17,0 g N,N-Di-cyclohexylcarbodiimid zugegeben und das Reaktionsgemisch wird für 3 Stunden bei Raumtemperatur gerührt. Das Reaktionsgemisch wird filtriert. Der abgetrennte Niederschlag wird verworfen, das Filtrat mit Wasser gewaschen und über Natriumsulfat getrocknet. Nach dem Eindampfen wird der ölige Rückstand durch Säulenchromatographie an Kieselgel mit Hexan/Essigester (9:1) gereinigt. Man erhält so 20,0 g 3-Butensäure-2-(4-phenoxy-phenxoy)-äthylester der Formel

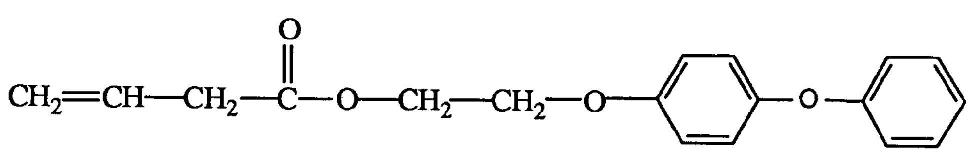

als farbloses Oel mit einem Brechungsindex von $n_D^{23}$: 1,5538.

b) 14,8 g 3-Butensäure-2-(4-phenoxy-phenxoy)-äthylester werden in 30 ml Diäthylenglykoldimethyläther (Diglyme) gelöst und unter Rühren auf +165°C erhitzt. Bei dieser Temperatur lässt man innerhalb von 8 Stunden eine Lösung von 16,9 g Natrium-dichlorfluoracetat in 30 ml Diglyme zutropfen. Das Gemisch wird für eine Stunde bei +165°C weitergerührt und anschliessend auf Raumtemperatur abgekühlt. Das Reaktionsgemisch wird filtriert, der Rückstand mit 20 ml Diglyme gewaschen, das Filtrat komplett eingedampft, danach 40 ml Diäthyläther aufgenommen und zweimal mit je 20 ml Wasser gewaschen und über Natriumsulfat getrocknet. Nach dem Eindampfen des Lösungsmittels wird der ölige Rückstand durch Säulenchromatographie an Kieselgel mit Hexan/Essigester (9:1) gereinigt. Man erhält so 12,5 g 2-(2-Chlor-2-fluorcyclopropyl)-essigsäure-2-(4-phenoxy-phenoxy)-äthylester als leicht gelbliches Oel mit einem Brechungsindex von $n_D^{20}$: 1,5461.

In analoger Weise können die in den folgenden Tabellen aufgelisteten Verbindungen der Formel I hergestellt werden.

Tabelle 1:

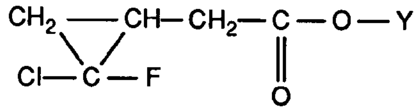

| Verb. Nr. | Y | physikalische Daten |
|---|---|---|
| 1.01 | 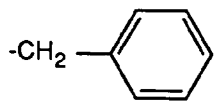 | $n_D^{20}$: 1,5031 |
| 1.02 | H | $n_D^{20}$: 1,4442 |
| 1.03 | 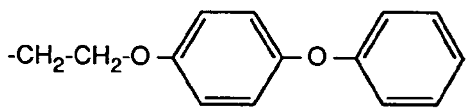 | $n_D^{20}$: 1,5461 |
| 1.04 | 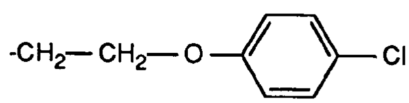 | $n_D^{20}$: 1,5168 |
| 1.05 | $CH_3$ | |
| 1.06 | $C_2H_5$ | |
| 1.07 | $C_3H_7$-n | |
| 1.08 | $C_3H_7$-i | |
| 1.09 | $C_4H_9$-t | |
| 1.10 | $C_4H_9$-n | |
| 1.11 | $C_6H_{13}$-n | |
| 1.12 | $C_{10}H_{21}$-n | |
| 1.13 | $C_{20}H_{41}$-n | |
| 1.14 | $CH_2C(CH_3)_3$ | $n_D^{23}$: 1,4330 |
| 1.15 | $CH_2CH_2OH$ | |
| 1.16 | $CH_2CH_2O$-$COCH_3$ | |
| 1.17 | $CH_2CH_2O$-$COOCH_3$ | |
| 1.18 | $CH_2CH_2OCONHC_6H_5$ | |
| 1.19 | $CH_2CH_2OC_2H_5$ | |
| 1.20 | $CH_2CH_2OCH_2CH_2OCH_3$ | |
| 1.21 | $CH_2CCl_3$ | |
| 1.22 | $CH_2CBr_3$ | |
| 1.23 | $CH_2CH_2SCH_3$ | |

| Verb. Nr. | Y | physikalische Daten |
|---|---|---|
| 1.24 | $CH_2CH_2OCH_2C_6H_5$ | |
| 1.25 | $CH_2CH_2Cl$ | |
| 1.26 | $CH_2$-$C_6H_5$ | |
| 1.27 | $CH(CH_3)C_6H_5$ | |
| 1.28 | $CH(CH_3)C_6H_5$ (R) | |
| 1.29 | $CH(CH_3)C_6H_5$ (S) | |
| 1.30 | $CH_2$-$C_6H_4$-$NO_2$-(4) | |
| 1.31 | $CH_2$-$C_6H_4$-$NO_2$-(2) | |
| 1.32 | $CH_2$-$C_6H_4$-Cl-(2) | |
| 1.33 | $CH_2$-$C_6H_5$-F-(4) | |
| 1.34 | $CH_2$-$C_6H_4$-O-$C_6H_5$-(3) | |
| 1.35 | -$C_6H_5$ | |
| 1.36 | -$C_6H_4$-O-$C_6H_5$-(4) | $n_D^{23}$: 1,5478 |
| 1.37 | -$C_6H_4$-$NO_2$-(4) | |
| 1.38 | -$C_6H_4$-F-(4) | |
| 1.39 | 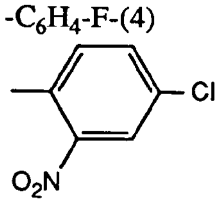 | |
| 1.40 | 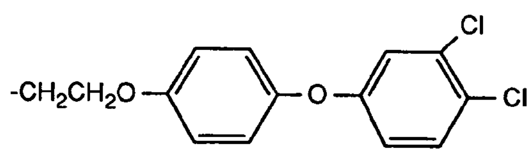 | |
| 1.41 | 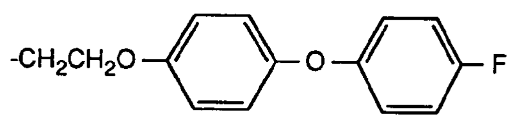 | |
| 1.42 | 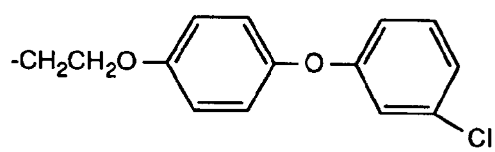 | |

| Verb. Nr. | Y | physikalische Daten |
| --- | --- | --- |
| 1.43 | 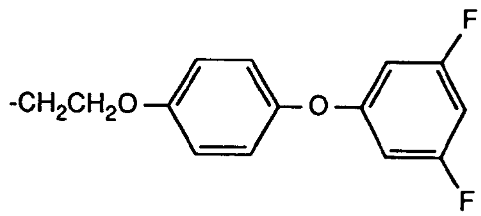 | |
| 1.44 | $C_6H_{11}$-cycl. | |
| 1.45 | $C_5H_9$-cycl. | |
| 1.46 | $C_3H_5$-cycl. | |
| 1.47 | 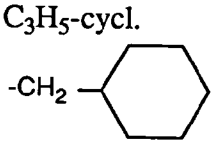 | |
| 1.48 | 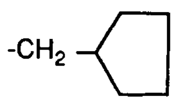 | |
| 1.49 | 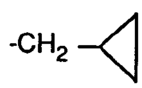 | |
| 1.50 | $-CH_2-CH{=}CH_2$ | |
| 1.51 | $-CH_2CH{=}C(CH_3)CH_2CH_2CH{=}C(CH_3)_2$ | |
| 1.52 | $-CH_2CH{=}C(CH_3)CH_2CH_2CH{=}C(CH_3)-CH_2CH_2CH{=}C(CH_3)_2$ | |
| 1.53 | $-CH_2-CH{\equiv}CH$ | |
| 1.54 | $-C_{16}H_{33}$-n | |
| 1.55 | $-CH(CH_2CH_2CH_2CH_3)_2$ | |
| 1.56 | $-CH_2-(CH_2)_4CH_2Cl$ | |
| 1.57 | 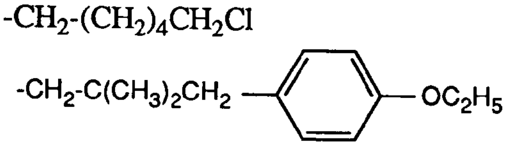 | |
| 1.58 | 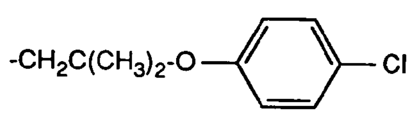 | |
| 1.59 | $-CH_2CH_2CH_2CH_2-C_6H_5$ | |
| 1.60 | 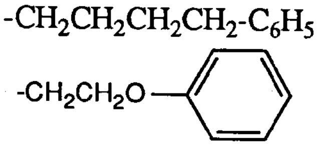 | |
| 1.61 | 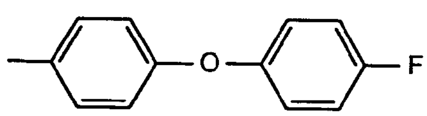 | |

| Verb. Nr. | Y | physikalische Daten |
|---|---|---|
| 1.62 | 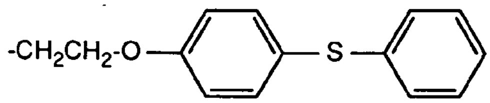 | $n_D^{22}$: 1,5744 |
| 1.63 | 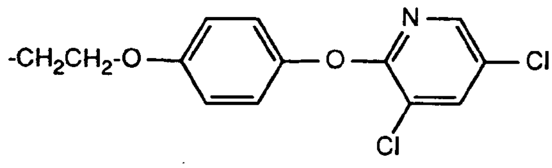 | |
| 1.64 | 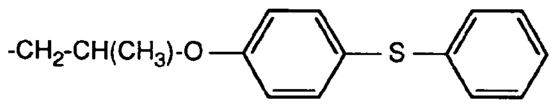 | |
| 1.65 | 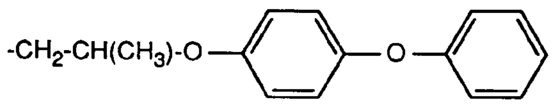 | |
| 1.66 | 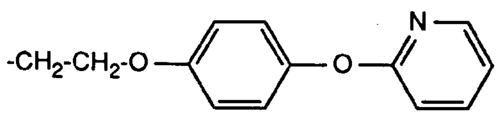 | $n_D^{22}$: 1,5449 |
| 1.67 | 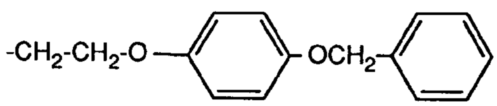 | Smp. 57-59°C |
| 1.68 | 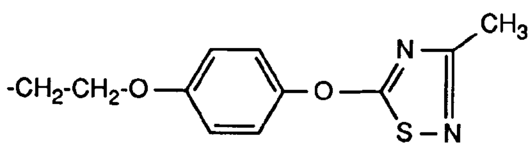 | |
| 1.69 | 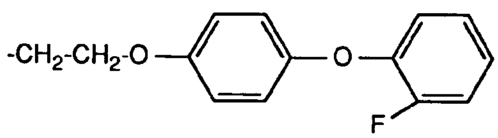 | |
| 1.70 | 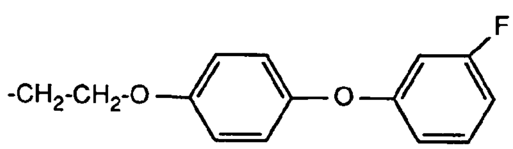 | |
| 1.71 | 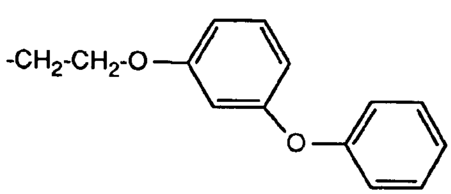 | |

| Verb. Nr. | Y | physikalische Daten |
|---|---|---|
| 1.72 | 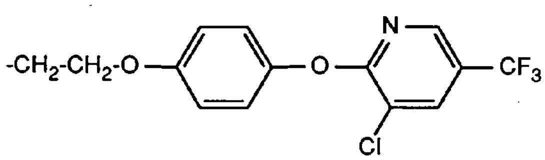 | |
| 1.73 | 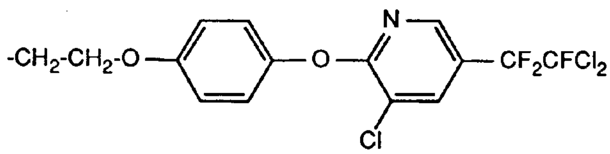 | |
| 1.74 | 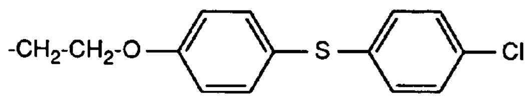 | |
| 1.75 | 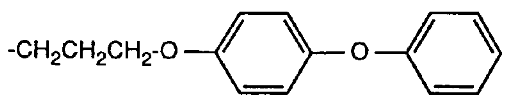 | |
| 1.76 | 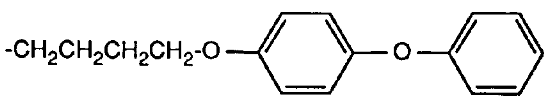 | |
| 1.77 | 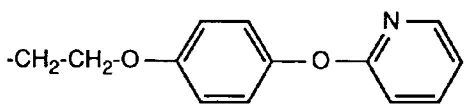 | |
| 1.78 | 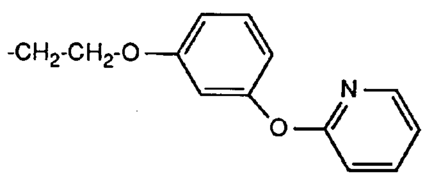 | |
| 1.79 | 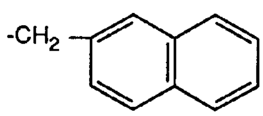 | |
| 1.80 | 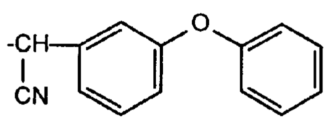 | |
| 1.81 | 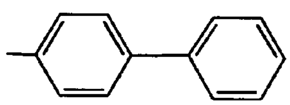 | |

| Verb. Nr. | Y | physikalische Daten |
|---|---|---|
| 1.82 | 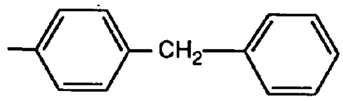 | |
| 1.83 | 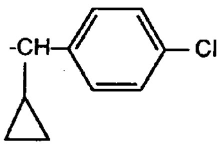 | |
| 1.84 | 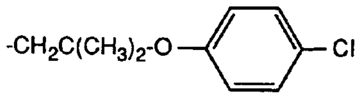 | |
| 1.85 | $-(CH_2)_2-C{\equiv}C-(CH_2)_4CH_3$ | |
| 1.86 | 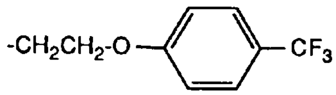 | |
| 1.87 | 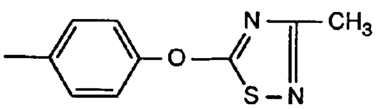 | $n_D^{22}$: 1,5472 |
| 1.88 | 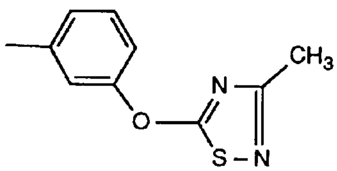 | |
| 1.89 | 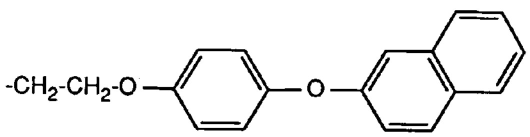 | |

Tabelle 2:

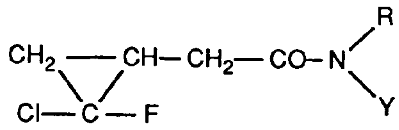

| Verb. Nr. | R | Y | physikalische Daten |
|---|---|---|---|
| 2.01 | H | $-CH_3$ | |
| 2.02 | $CH_3$ | $-CH_3$ | |
| 2.03 | $C_6H_{13}$-n | $-C_6H_{13}$-n | |
| 2.04 | H | $-C_6H_4$-F-(4) | |
| 2.05 | H | $-C_2H_5$ | |
| 2.06 | H | $-C_3H_7$-n | |
| 2.07 | H | $-C_3H_7$-i | |
| 2.08 | H | $-C_4H_9$-n | |
| 2.09 | H | $-C_4H_9$-t | |
| 2.10 | H | $-C_6H_{13}$-n | |
| 2.11 | H | $-C_{10}H_{21}$-n | |
| 2.12 | H | $-C_{20}H_{41}$-n | |
| 2.13 | H | $-CH_2C(CH_3)_3$ | |
| 2.14 | H | $-CH_2CH_2OH$ | |
| 2.15 | H | $-CH_2CH_2O-COC_6H_5$ | |
| 2.16 | H | $-CH_2CH_2O-COOCH_3$ | |
| 2.17 | H | $-CH_2CH_2O-CONHC_6H_5$ | |
| 2.18 | H | $-CH_2CH_2OC_2H_5$ | |
| 2.19 | H | $-CH_2CF_3$ | |
| 2.20 | H | $-CH_2CCl_3$ | |
| 2.21 | H | $-CH(CH_3)C_6H_5$ | |
| 2.22 | H | $-CH(CH_3)C_6H_5$ (R) | |
| 2.23 | H | $-CH(CH_3)C_6H_5$ (S) | |
| 2.24 | H | $-CH_2-C_6H_5$ | Smp. 50-52°C |
| 2.25 | H | $-CH_2-C_6H_4-NO_2$-(4) | |
| 2.26 | H | $-CH_2-C_6H_4$-F-(4) | |
| 2.27 | H | $-CH_2-C_6H_4$-Cl-(2) | |

| Verb. Nr. | R | Y | physikalische Daten |
|---|---|---|---|
| 2.28 | H | $-C_6H_5$ | |
| 2.29 | H | $-C_6H_4-NO_2-(4)$ | |
| 2.30 | H | 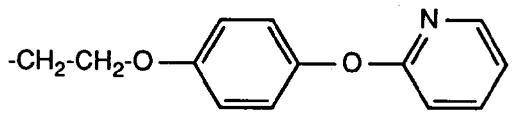 | |
| 2.31 | H | 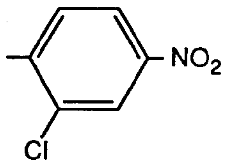 | |
| 2.32 | H | 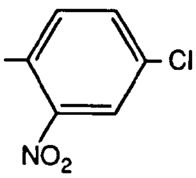 | |
| 2.33 | $CH_3$ | 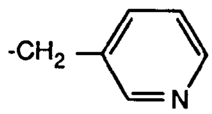 | |
| 2.34 | $CH_3$ | 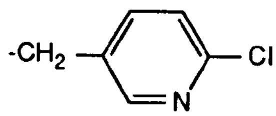 | |
| 2.35 | H | 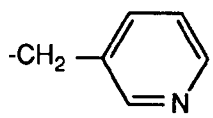 | |
| 2.36 | H | $-C_6H_4-CN-(4)$ | |
| 2.37 | H | $-C_6H_4-CF_3-(2)$ | |
| 2.38 | H | $-C_6H_4-C_6H_5-(4)$ | |
| 2.39 | H | $-C_6H_4-CF_3-(3)$ | |
| 2.40 | H | 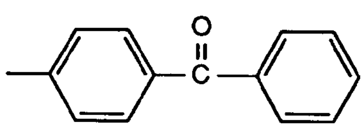 | |
| 2.41 | H | 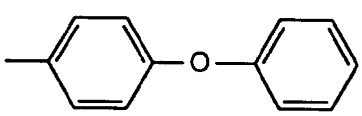 | |
| 2.42 | H | $-C_6H_4-Cl-(2)$ | |

| Verb. Nr. | R | Y | physikalische Daten |
|---|---|---|---|
| 2.43 | H | $-C_6H_4-Cl-(4)$ | Smp. 126-128°C |
| 2.44 | H | $-C_6H_4-Cl-(3)$ | |
| 2.45 | H | $-C_6H_4-OCH_3-(4)$ | |
| 2.46 | H | $-C_6H_4-CH_3-(4)$ | |
| 2.47 | H | $-C_6H_4-SCH_3-(3)$ | |
| 2.48 | H | H | |
| 2.49 | H | $-C_{16}H_{33}-n$ | |
| 2.50 | H | $-C_{18}H_{37}-n$ | |
| 2.51 | H | $-C_{12}H_{25}-n$ | |
| 2.52 | H | $-C_9H_{19}-n$ | |
| 2.53 | H | $-C_8H_{17}-n$ | |
| 2.54 | H | $-CH_2(CH_3)-CH_2CH_2CH_3$ | |
| 2.55 | H | $-CH_2CH_2CH_2CH_2OH$ | |
| 2.56 | H | $-CH_2CH_2CH_2CH_2OCH_3$ | |
| 2.57 | H | 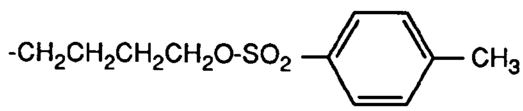 | |
| 2.58 | H | $-CH_2CH_2CH_2CH_2O-SO_2CH_3$ | |
| 2.59 | H | 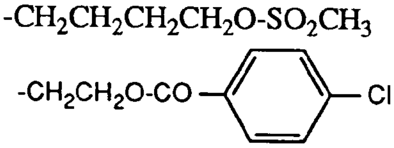 | |
| 2.60 | H | $-CH_2CH_2O-CH_2CF_3$ | |
| 2.61 | H | $-CH_2CH_2N(CH_3)_2$ | |
| 2.62 | H | $-CH_2(CH_3)-CH_2CH_2CH_2-N(CH_2CH_3)_2$ | |
| 2.63 | H | 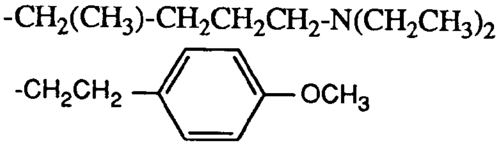 | |
| 2.64 | H | 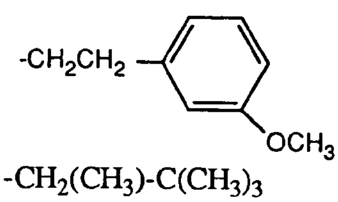 | |
| 2.65 | H | $-CH_2(CH_3)-C(CH_3)_3$ | |
| 2.66 | H | $-CH_2CH(OCH_2CH_3)_2$ | |
| 2.67 | H | $-CH_2-CO-C_6H_5$ | |

| Verb. Nr. | R | Y | physikalische Daten |
|---|---|---|---|
| 2.68 | H | 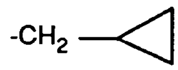 | |
| 2.69 | H | 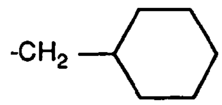 | |
| 2.70 | H | $-CH_2(CH_3)-CH_2OCH_3$ | |
| 2.71 | H | $-CH_2CH_2-CH(CH_3)_2$ | |
| 2.72 | H | $-CH_2(CH_2)_7-CH=CH-(CH_2)_7CH_3$ | |
| 2.73 | $CH_3$ | $-C_{18}H_{37}$-n | |
| 2.74 | $CH_3$ | $-C_3H_7$-n | |
| 2.75 | $CH_3$ | $-CH_2CH_2-C_6H_5$ | |
| 2.76 | H | $-CH_2-C_6H_4-OCH_3$-(4) | |
| 2.77 | H | $-CH_2-C_6H_4-F$-(2) | |
| 2.78 | H | $-CH_2-C_6H_3-F_2$-(2,6) | |
| 2.79 | H | $-CH_2-C_6H_3-F_2$-(2,4) | |
| 2.80 | H | $-CH_2-C_6H_3-F_2$-(3,4) | |
| 2.81 | H | $-CH_2-C_6H_4-Cl$-(4) | |
| 2.82 | H | $-CH_2-C_6H_3-Cl_2$-(3,4) | |
| 2.83 | H | $-CH_2-C_6H_4-CF_3$-(4) | |
| 2.84 | H | $-CH_2-C_6H_4-NO_2$-(3) | |
| 2.85 | H | $-CH(C_6H_5)_2$ | |
| 2.86 | H | 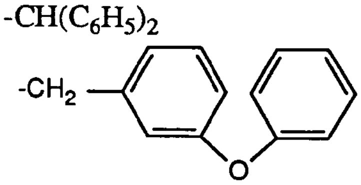 | |
| 2.87 | H | 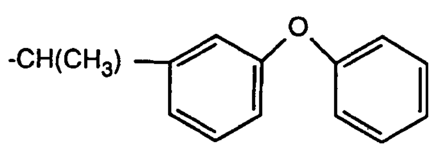 | |
| 2.88 | H | 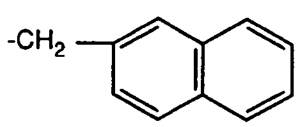 | |

| Verb. Nr. | R | Y | physikalische Daten |
|---|---|---|---|
| 2.89 | H | 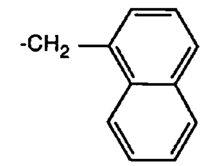 | |
| 2.90 | H | 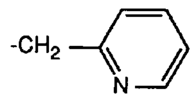 | $n_D^{23}$: 1,5297 |
| 2.91 | H | 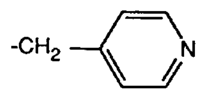 | |
| 2.92 | H | 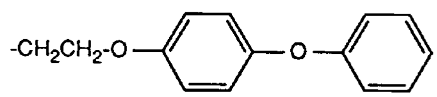 | Smp. 49-51°C |
| 2.93 | H | 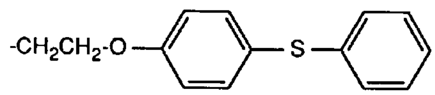 | Smp. 40-43°C |
| 2.94 | $CH_3$ | 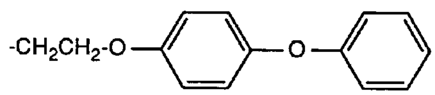 | |
| 2.95 | H | 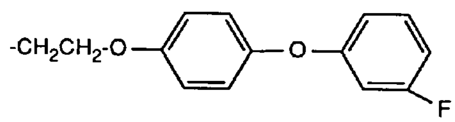 | |
| 2.96 | H | 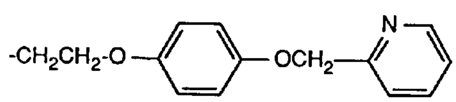 | |
| 2.97 | H | 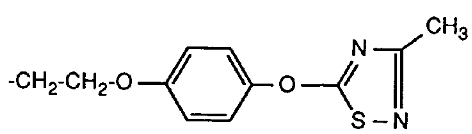 | |
| 2.98 | H | 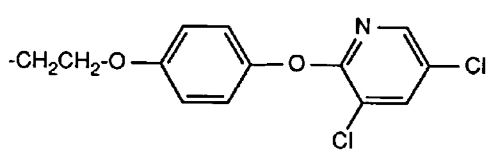 | |

| Verb. Nr. | R | Y | physikalische Daten |
|---|---|---|---|
| 2.99 | H | 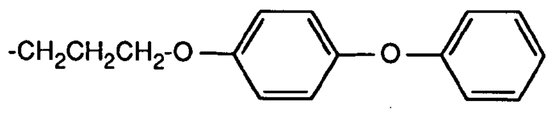 | Smp. 58-60°C |
| 2.100 | H | 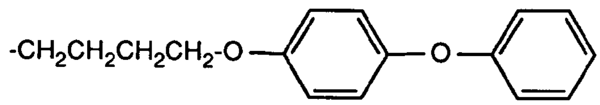 | |
| 2.101 | H | 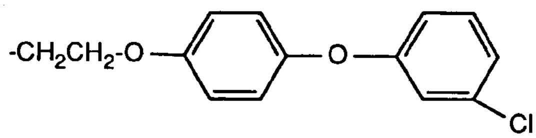 | |
| 2.102 | H | $-C_6H_4-OCF_3-(4)$ | $n_D^{22}$: 1,4904 |
| 2.103 | H | $-C_6H_4-Si(CH_3)_3-(4)$ | Smp. 94-96°C |

Formulierungsbeispiele (% = Gewichtsprozent)

| Beispiel F1: Emulsions-Konzentrate | a) | b) | c) |
|---|---|---|---|
| Wirkstoff Nr. 1.04 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.03 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.03 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.03 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 2.43 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Biespiel F6: Emulsions-Konzentrat

| | |
|---|---|
| Wirkstoff Nr. 2.43 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten konzentration hergestellt werden.

| Beispiel F7: Stäubemittel | a) | b) |
|---|---|---|
| Wirkstoff Nr. 2.43 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff Nr. 2.103 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff Nr. 2.43 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Biespiel F10: Suspensions-Konzentrat

| | |
|---|---|
| Wirkstoff Nr. 2.43 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Boophilus microplus

Vollgesogene adulte Zecken Weibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Verbindungen gemäss Tabellen 1 und 2 zeigen in diesem Test gute Wirkung gegen Boophilus microplus. Insbesondere die Verbindungen 1.01, 1.02, 1.03, 1.04, 1.62, 2.43, 2.102 und 2.103 zeigen eine Wirkung über 80 %.

Beispiel B2: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Nilaparvata lugens in diesem Test. Insbesondere die Verbindungen 1.02, 1.03, 1.04, 1.62, 2.102, 2.103 und 2.024 zeigen eine Wirkung über 80 %.

Beispiel B3: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt. Verbindungen der Tabellen 1 und 2 zeigen eine gute Wirkung gegen Tetranychus urticae in diesem Test. Insbesondere die Verbindungen 1.01, 1.02, 1.03, 1.04, 1.62, 1.66, 2.24, 2.43, 2.102 und 2.103 zeigen eine Wirkung über 80 %.

Beispiel B4: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Aphis craccivora in diesem Test. Insbesondere die Verbindungen 1.01, 1.02, 1.03, 1.04, 1.62, 1.66, 2.24, 2.43 und 2.103 zeigen eine Wirkung über 80 %.

Beispiel B5: Systemische Wirkung gegen Nilaparvata lugens

Töpfe mit Reispflanzen werden in eine wässrige EmulsionsLösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Verbindungen der Tabellen 1 und 2 zeigen eine gute Wirkung gegen Nilaparvata lugens in diesem Test. Insbesondere die Verbindungen 1.01, 2.24 und 2.103 zeigen eine Wirkung über 80 %.

Beispiel B6: Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben ermittelt. Verbindungen gemäss Tabellen 1 und 2 zeigen gute Wirkung gegen Dermanyssus gallinae in diesem Test. Insbesondere die Verbindungen 1.01, 1.03 und 1.04 zeigen eine Wirkung über 80 %.

Beispiel B7: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (% Reduktion der Population).

Verbindungen gemäss Tabellen 1 und 2 zeigen in diesem Test gute Wirkung gegen Heliothis virescens. Insbesondere die Verbindungen 1.01, 1.04, 1. 14 und 2.43 zeigen eine Wirkung über 80 %.

## Patentansprüche

**1.** 2-(2-Chlor-2-fluorcyclopropyl)-essigsäurederivate der Formel I

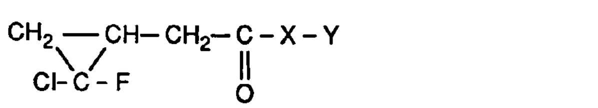

(I)

worin

X Sauerstoff oder -NR-,

Y Wasserstoff oder jeweils unsubstituiertes oder substituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl, und

R Wasserstoff oder $C_1$-$C_6$-Alkyl bedeuten.

2. Verbindungen gemäss Anspruch 1, worin Y für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Aryl, $C_3$-$C_{20}$-Halogenalkenyl, $C_3$-$C_{20}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyan, Benzoyl, Halogenbenzoyl, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, Phenylthio oder Halogenphenylthio substituiertes Aryl; oder durch Hydroxy, Halogen, Di-$C_1$-$C_4$-alkylamino, $C_1$-$C_4$-Alkoxy, $C_1$-$C_4$-Halogenalkoxy, Cyan, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_1$-$C_6$-Alkoxycarbonyloxy, $C_3$-$C_7$-Cycloalkyl, Aryl, Aryloxy, Anilinocarbonyloxy, Benzyloxy, Arylcarbonyloxy, Arylthio, Arylsulfonyl, Arylsulfinyl, Arylsulfonyloxy, Arylcarbonyl oder Pyridyl substituiertes $C_1$-$C_{20}$-Alkyl; wobei die Aryl- und Pyridylgruppen jeweils durch Halogen, $C_1$-$C_4$-Alkyl, $C_1$-$C_4$-Halogenyl, $C_1$-$C_4$-Alkoxy, $C_1$-$C_4$-Halogenalkoxy, $C_1$-$C_4$-Alkylthio, Nitro, Benzyloxy, Cyan, Phenoxy, Halogenphenoxy, Pyridyloxy, Halogenpyridyloxy, $C_1$-$C_4$-Halogenalkylpyridyloxy, $C_1$-$C_4$-Alkylthiadiazolyloxy, Phenylthio oder Halogenphenylthio substituiert sein können, steht.

3. Verbindungen gemäss Anspruch 1, dadurch gekennzeichnet, dass X Sauerstoff, -NH-, -$NCH_3$- oder -$NC_2H_5$ bedeutet.

4. Verbindungen gemäss Anspruch 1, durch gekennzeichnet, dass Y für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Phenyl, Naphthyl, $C_3$-$C_{20}$-Halogenalkenyl, $C_3$-$C_{20}$-Halogenalkinyl; durch Fluor, Chlor, Brom oder $C_1$-$C_3$-Alkyl substituiertes $C_3$-$C_7$-Cycloalkyl; durch Fluor, Chlor, Brom oder $C_1$-$C_3$-Alkyl, $C_1$-$C_3$-Halogenalkyl, $C_1$-$C_3$-Alkoxy, $C_1$-$C_3$-Halogenaloxy, $C_1$-$C_3$-Alkylthio, Nitro, Cyan, Phenoxy, Halogenphenoxy, Phenylthio oder Halogenphenylthio substituiertes Phenyl oder Naphthyl, oder durch Hydroxy, Fluor, Chlor, Brom, Di-$C_1$-$C_4$alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Phenyl, Phenoxy, Phenylthio, Phenylsulfonyloxy oder Pyridyl substituiertes $C_1$-$C_{20}$-Alkyl, wobei die Phenyl- und Pyridylgruppen jeweils durch Fluor, Chlor, Brom, $C_1$-$C_3$-Alkyl, $C_1$ -$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiert sein können, steht.

5. Verbindungen gemäss Anspruch 3, dadurch gekennzeichnet, dass X Sauerstoff oder NH bedeutet.

6. Verbindungen gemäss Anspruch 4, dadurch gekennzeichnet, dass Y Phenyl, Benzyl, Naphthyl, 3-Pyridylmethyl, oder jeweils durch Fluor, Chlor, Brom, $C_1$-$C_3$-Alkyl, $C_1$ -$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl, Benzyl, Naphthyl oder 3-Pyridylmethyl bedeutet.

7. Verbindungen gemäss Anspruch 4, dadurch gekennzeichnet, dass Y $C_1$-$C_{12}$-Alkyl oder durch Hydroxy, Fluor, Chlor, Brom, Dimethylamino, Methoxy, Aethoxy, Methoxyäthoxy, Aethoxyäthoxy, Methylthio, Aethylthio, Cyclopropyl, Cyclopentyl, Cyclohexyl, Phenyl oder Phenoxy substituiertes $C_1$-$C_{12}$-Alkyl bedeutet, wobei der Phenyl- oder Phenoxyrest jeweils durch Fluor, Chlor, Brom, Phenoxy, Halogenphenoxy oder Phenylthio substituiert sein kann.

8. Verbindungen gemäss Anspruch 4, dadurch gekennzeichnet, dass Y $C_3$-$C_{12}$-Alkinyl, $C_3$-$C_{12}$-Alkenyl oder jeweils durch Fluor, Chlor oder Brom substituiertes $C_3$-$C_{12}$-Alkenyl oder $C_3$-$C_{12}$-Alkinyl bedeutet.

9. Verbindungen gemäss Anspruch 7, dadurch gekennzeichnet, dass X Sauerstoff oder NR und R Wasserstoff oder $C_1$-$C_4$-Alkyl bedeutet.

**10.** Verbindungen gemäss Anspruch 1, dadurch gekennzeichnet, dass der Rest Y das Formelfragment

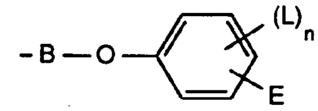

bedeutet, worin

B für eine $C_2$-$C_6$-Alkylenbrücke,

L für Halogen oder Methyl,

n für die Zahl null, eins oder zwei und

E für Benzyloxy, Phenyl, Halogenphenoxy, Naphthoxy, Pyridyloxy, Halogenpyridyloxy, $C_1$-$C_4$-Halogenalkylpyridyloxy, $C_1$-$C_4$-Alkylthiadiazolyloxy, Phenylthio oder Halogenphenylthio stehen.

**11.** Verbindungen gemäss Anspruch 1, dadurch gekennzeichnet, dass X für Sauerstoff und der Rest Y für das Formelfragment

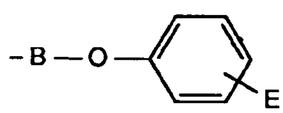

stehen, worin

B eine Brücke 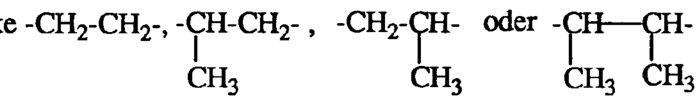 , oder und

E Phenoxy, Phenylthio oder Halogenphenoxy bedeuten.

**12.** Eine Verbindung der Formel 1, ausgewählt aus der Gruppe 2-(2-Chlor-2-fluorcyclopropyl)-essigsäure, 2-(2-Chlor-2-fluorcyclopropyl)-essigsäurebenzylester, 2-(2-Chlor-2-fluorcyclopropyl)-essigsäure-2-(4-chlor-phenoxy)-äthylester, 2-(2-Chlor-2-fluorcyclopropyl)-essigsäure-2-(4-phenoxy-phenoxy)-äthylester und 2-(2-Chlor-2-fluorcyclopropyl)-essigsäure-2-(4-phenylthio-phenoxy)-äthylester.

**13.** Verfahren zur Herstellung der Verbindungen der Formel I, gemäss Anspruch 1, dadurch gekennzeichnet, dass man entweder

a) ein 2-(2-Chlor-2-fluorcyclopropyl)-essigsäurehalogenid der Formel II

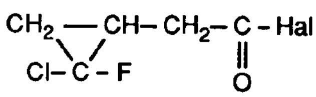

(II)

worin Hal für Chlor oder Brom steht, gegebenenfalls in einem inerten Lösungsmittel und in Gegenwart eines säurebindenden Mittels mit einem Alkohol oder Arnin der Formel III

H-X-Y (III)

worin X und Y die unter Formel I gegebenen Bedeutungen haben, umsetzt, oder

b) ein 3-Butensäurederivat der Formel IV

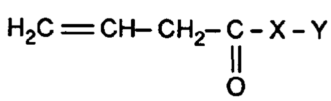

(IV)

worin X und Y die unter Formel I gegebenen Bedeutungen haben, in einem inerten Lösungsmittel mit Chlorfluorcarben umsetzt, oder

c) die freie 2-(2-Chlor-2-fluorcyclopropyl)-essigsäure der Formel Ia

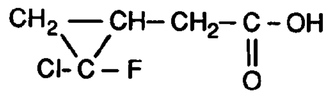

(Ia)

gegebenenfalls in Gegenwart eines inerten Lösungsmittels und eines Katalysators oder eines wasserentziehenden Mittels mit einem Alkohol oder Amin der Formel III umsetzt.

**14.** Schädlingsbekämpfungsmittel, welches als aktive Komponente mindestens eine Verbindung der Formel I nach Anspruch 1 enthält.

**15.** Mittel gemäss Anspruch 14, dadurch gekennzeichnet, dass es zusätzlich noch mindestens einen Träger enthält.

**16.** Verwendung einer Verbindung der Formel I nach Anspruch 1 zur Bekämpfung von Schädlingen an Tieren und Pflanzen.

**17.** Verwendung gemäss Anspruch 16, dadurch gekennzeichnet, dass es sich bei den Schädlingen um pflanzenschädigende Insekten und Arachniden handelt.

**18.** Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch 1 behandelt.

**19.** 2-(2-Chlor-2-fluorcyclopropyl)-essigsäurehalogenid der Formel II

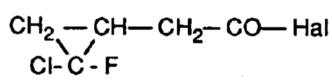

(II)

worin Hal für Chlor oder Brom steht.

**Patentansprüche für folgenden Vertragsstaat : ES**

**1.** Verfahren zur Herstellung der 2-(2-Chlor-2-fluorcyclopropyl)-essigsäurederivate der Formel I

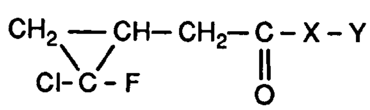

(I)

worin
X Sauerstoff oder -NR-,
Y Wasserstoff oder jeweils unsubstituiertes oder substituiertes $C_1$-$C_{20}$-Alkyl, $C_3$-$C_7$-Cycloalkyl, $C_3$-$C_{20}$-Alkenyl, $C_3$-$C_{20}$-Alkinyl, Benzyl oder Aryl, und
R Wasserstoff oder $C_1$-$C_6$-Alkyl bedeuten, dadurch gekennzeichnet, dass man entweder
a) ein 2-(2-Chlor-2-fluorcyclopropyl)-essigsäurehalogenid der Formel II

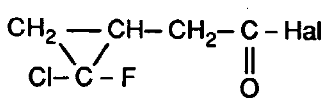

(II)

worin Hal für Chlor oder Brom steht, gegebenenfalls in einem inerten Lösungsminel und in Gegenwart

eines säurebindenden Mittels mit einem Alkohol oder Amin der Formel III

H-X-Y (III)

worin X und Y die unter Formel I gegebenen Bedeutungen haben, umsetzt, oder

b) ein 3-Butensäurederivat der Formel IV

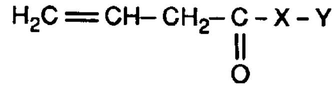 (IV)

worin X und Y die unter Formel I gegebenen Bedeutungen haben, in einem inerten Lösungsmittel mit Chlorfluorcarben umsetzt, oder

c) die freie 2-(2-Chlor-2-fluorcyclopropyl)-essigsäure der Formel Ia

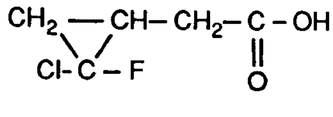 (Ia)

gegebenenfalls in Gegenwart eines inerten Lösungsmittels und eines Katalysators oder eines wasserentziehenden Mittels mit einem Alkohol oder Amin der Formel III umsetzt.

2. Verfahren gemäss Anspruch 1, worin Y für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Aryl, $C_3$-$C_{20}$-Halogenalkenyl, $C_3$-$C_{20}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyan, Benzoyl, Halogenbenzoyl, Phenoxy, Halogenphenoxy, $C_1$-$C_4$-Alkylphenoxy, $C_1$-$C_4$-Halogenalkylphenoxy, Tri-$C_1$-$C_4$-alkylsilyl, N-Pyrrolidinyl, N-Piperidinyl, N-Pyrrolidin-2-onyl, N-Piperidin-2-onyl, $C_1$-$C_4$-Alkylamino, Di-$C_1$-$C_4$-alkylamino, Anilino, N-$C_1$-$C_4$-Alkylanilino, N-Formylanilino, Phenylthio oder Halogenphenylthio substituiertes Aryl; oder durch Hydroxy, Halogen, Di-$C_1$-$C_4$alkylamino, $C_1$-$C_4$-Alkoxy, $C_1$-$C_4$-Halogenalkoxy, Cyan, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_1$-$C_6$-Alkoxycarbonyloxy, $C_3$-$C_7$-Cycloalkyl, Aryl, Aryloxy, Anilinocarbonyloxy, Benzyloxy, Arylcarbonyloxy, Arylthio, Arylsulfonyl, Arylsulfinyl, Arylsulfonyloxy, Arylcarbonyl oder Pyridyl substituiertes $C_1$-$C_{20}$-Alkyl; wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Benzyloxy, Cyan, Phenoxy, Halogenphenoxy, Pyridyloxy, Halogenpyridyloxy, $C_1$-$C_4$-Halogenalkylpyridyloxy, $C_1$-$C_4$-Alkylthiadiazolyloxy, Phenylthio oder Halogenphenylthio substituiert sein können, steht.

3. Verfahren gemäss Anspruch 1, dadurch gekennzeichnet, dass X Sauerstoff, -NH-, $-NCH_3$- oder $-NC_2H_5$ bedeutet.

4. Verfahren gemäss Anspruch 1, dadurch gekennzeichnet, dass Y für Wasserstoff, $C_1$-$C_{20}$-Alkyl, $C_3$-$C_7$-Cycloalkyl, $C_3$-$C_{20}$-Alkenyl, $C_3$-$C_{20}$-Alkinyl, Phenyl, Naphthyl, $C_3$-$C_{20}$-Halogenalkenyl, $C_3$-$C_{20}$-Halogenalkinyl; durch Fluor, Chlor, Brom oder $C_1$-$C_3$-Alkyl substituiertes $C_3$-$C_7$-Cycloalkyl; durch Fluor, Chlor, Brom oder $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl oder Naphthyl, oder durch Hydroxy, Fluor, Chlor, Brom, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Phenyl, Phenoxy, Phenylthio, Phenylsulfonyloxy oder Pyridyl substituiertes $C_1$-$C_{20}$-Alkyl, wobei die Phenyl- und Pyridylgruppen jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiert sein können, steht.

5. Verfahren gemäss Anspruch 3, dadurch gekennzeichnet, dass X Sauerstoff oder NH bedeutet.

6. Verfahren gemäss Anspruch 4, dadurch gekennzeichnet, dass Y Phenyl, Benzyl, Naphthyl, 3-Pyridylmethyl, oder jeweils durch Fluor, Chlor, Brom, $C_1$-$C_3$-Alkyl, $C_1$-$C_3$-Halogenalkyl, $C_1$-$C_3$-Alkoxy, $C_1$-$C_3$-Halogenalkoxy, $C_1$-$C_3$-Alkylthio, Nitro, Cyan, Phenoxy, Halogenphenoxy, Phenylthio oder Halogenphenylthio substituiertes Phenyl, Benzyl, Naphthyl oder 3-Pyridylmethyl bedeutet.

**7.** Verfahren gemäss Anspruch 4, dadurch gekennzeichnet, dass Y $C_1$-$C_{12}$-Alkyl oder durch Hydroxy, Fluor, Chlor, Brom, Dimethylamino, Methoxy, Aethoxy, Methoxyäthoxy, Aethoxyäthoxy, Methylthio, Aethylthio, Cyclopropyl, Cyclopentyl, Cyclohexyl, Phenyl oder Phenoxy substituiertes $C_1$-$C_{12}$-Alkyl bedeutet, wobei der Phenyl- oder Phenoxyrest jeweils durch Fluor, Chlor, Brom, Phenoxy, Halogenphenoxy oder Phenylthio substituiert sein kann.

**8.** Verfahren gemäss Anspruch 4, dadurch gekennzeichnet, dass Y $C_3$-$C_{12}$-Alkinyl, $C_3$-$C_{12}$-Alkenyl oder jeweils durch Fluor, Chlor oder Brom substituiertes $C_3$-$C_{12}$-Alkenyl oder $C_3$-$C_{12}$-Alkinyl bedeutet.

**9.** Verfahren gemäss Anspruch 7, dadurch gekennzeichnet, dass X Sauerstoff oder NR und R Wasserstoff oder $C_1$-$C_4$-Alkyl bedeutet.

**10.** Verfahren gemäss Anspruch 1, dadurch gekennzeichnet, dass der Rest Y das Formelfragment

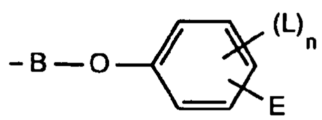

bedeutet, worin
B für eine $C_2$-$C_6$-Alkylenbrücke,
L für Halogen oder Methyl,
n für die Zahl null, eins oder zwei und
E für Benzyloxy, Phenyl, Halogenphenoxy, Naphthoxy, Pyridyloxy, Halogenpyridyloxy, $C_1$-$C_4$-Halogenalkylpyridyloxy, $C_1$-$C_4$-Alkylthiadiazolyloxy, Phenylthio oder Halogenphenylthio stehen.

**11.** Verfahren gemäss Anspruch 1, dadurch gekennzeichnet, dass X für Sauerstoff und der Rest Y für das Formelfragment

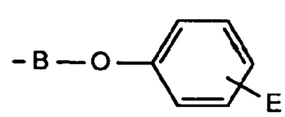

stehen, worin

B eine Brücke $-CH_2-CH_2-$, $-CH(CH_3)-CH_2-$, $-CH_2-CH(CH_3)-$ oder $-CH(CH_3)-CH(CH_3)-$ und

E Phenoxy, Phenylthio oder Halogenphenoxy bedeuten.

**12.** Verfahren gemäss Anspruch 1 zur Herstellung einer Verbindung der Formel I, ausgewählt aus der Gruppe 2-(2-Chlor-2-fluorcyclopropyl)-essigsäure, 2-(2-Chlor-2-fluorcyclopropyl)-essigsäurebenzylester, 2-(2-Chlor-2-fluorcyclopropyl)-essigsäure-2-(4-chlorphenoxy)-äthylester, 2-(2-Chlor-2-fluorcyclopropyl)-essigsäure-2-(4-phenoxy-phenoxy)-äthylester und 2-(2-Chlor-2-fluorcyclopropyl)- essigsäure-2- (4-phenylthio-phenoxy)-äthylester.

**13.** Schädlingsbekämpfungsmittel, welches als aktive Komponente mindestens eine Verbindung der Formel I nach Anspruch 1 enthält.

**14.** Mittel gemäss Anspruch I3, dadurch gekennzeichnet, dass es zusätzlich noch mindestens einen Träger

enthält.

**15.** Verwendung einer Verbindung der Formel I nach Anspruch 1 zur Bekämpfung von Schädlingen an Tieren und Pflanzen.

**16.** Verwendung gemäss Anspruch 15, dadurch gekennzeichnet, dass es sich bei den Schädlingen um pflanzenschädigende Insekten und Arachniden handelt.

**17.** Verfahren zur Bekämpfung von tier- und pflanzenschädigenden Insekten und Arachniden, dadurch gekennzeichnet, dass man die Schädlinge oder ihren Lebensraum mit einer wirksamen Menge einer Verbindung der Formel I gemäss Anspruch 1 behandelt.

**18.** Verfahren zur Herstellung des 2-(2-Chlor-2-fluorcyclopropyl)-essigsäurehalogenids der Formel II

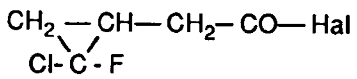

(II)

worin Hal für Chlor oder Brom steht, dadurch gekennzeichnet, dass man einen Niederalkylester der Formel Ib

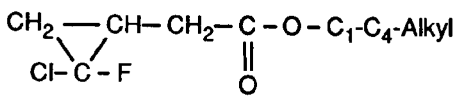

(Ib)

hydrolysiert und halogeniert.